# EUROPEAN PATENT SPECIFICATION

(11) **EP 2 919 818 B1**
(45) Date of publication and mention of the grant of the patent: **07.02.2018**
(21) Application number: 13820914.3
(22) Date of filing: 28.10.2013
(51) Int. Cl.: A61K 49/00

(54) **KIT FOR DIAGNOSING GLUTEN SENSITIVITY**
KIT FÜR DIAGNOSING GLUTEN EMPFINDLICHKEIT
KIT POUR DIAGNOSTIQUER LA SENSIBILITÉ AU GLUTEN

(30) Priority: 15.11.2012 IT MI20121932
(43) Date of publication of application: 23.09.2015
(73) Proprietor: Fondazione IRCCS "CA' GRANDA - OSPEDALE MAGGIORE POLICLINICO", 20122 Milan (IT)
(72) Inventor: ELLI, Luca, I-20131 Milano (IT); TOMBA, Carolina, I-20137 Milano (IT); RONCORONI, Leda, I-20124 Milano (IT); BARDELLA, Maria Teresa, I-20136 Milano (IT)
(74) Representative: Biggi, Cristina
(86) International application number: PCT/IB2013/059721
(87) International publication number: WO 2014/076602

(56) References cited:
- BIESIEKIERSKI JESSICA R ET AL: "Gluten Causes Gastrointestinal Symptoms in Subjects Without Celiac Disease: A Double-Blind Randomized Placebo-Controlled Trial", AMERICAN JOURNAL OF GASTROENTEROLOGY, vol. 106, no. 3, March 2011 (2011-03), pages 508-514, XP002701058,
- NIVELONI S ET AL: "Gluten sensitivity in patients with primary biliary cirrhosis", AMERICAN JOURNAL OF GASTROENTEROLOGY 199803 US, vol. 93, no. 3, March 1998 (1998-03), pages 404-408, XP002701059, ISSN: 0002-9270
- LEFFLER D ET AL: "Kinetics of the histological, serological and symptomatic responses to gluten challenge in adults with coeliac disease", GUT 2013 BMJ PUBLISHING GROUP GBR, vol. 62, no. 7, 22 May 2012 (2012-05-22), pages 996-1004, XP008163476, ISSN: 0017-5749
- ANTONIO CARROCCIO ET AL: "Non-Celiac Wheat Sensitivity Diagnosed by Double-Blind Placebo-Controlled Challenge: Exploring a New Clinical Entity", AMERICAN JOURNAL OF GASTROENTEROLOGY, vol. 107, no. 12, 24 July 2012 (2012-07-24), pages 1898-1906, XP055306111, US ISSN: 0002-9270, DOI: 10.1038/ajg.2012.236

## Description

The present invention relates to a kit for use in a method for determining whether a subject is sensitive to gluten as defined in the claims. Gluten is a very compact, elastic, porous lipoprotein substance present in wheat, barley, rye and several other graminaceous plants. The protein fraction of gluten contains gliadin and glutenin, which represent the toxic component of gluten. In fact, these proteins comprise the amino acid proline, in particular glutamine-proline repetitions, which are not easily digested by the human body.

Following the intake of gluten by a sensitive subject, as a result of intolerance, allergy or sensitivity to this substance, pathological conditions may arise which, despite manifesting roughly the same signs and the same symptoms, are clinically very different.

Celiac disease is a genetically based gluten intolerance associated with the markers DQ2 and DQ8 of the HLA system. This pathology manifests itself with immune-mediated damage to the intestinal mucosa and the appearance of specific serological markers, in particular anti-transglutaminase (tTG) antibodies, IgA class anti-endomysial antibodies (EMA) and IgA and IgG class anti-deamidated gliadin antibodies (AGA). The association between celiac disease and these specific antibodies is at the basis of the diagnostic technique of this pathology, which is in fact diagnosed by means of serological assays of the tTG, EMA and AGA antibodies.

Gluten allergy (also known wheat allergy) is a violent reaction of IgE to gliadin. This pathology manifests itself clinically with respiratory tract problems or anaphylactic reactions. At presence, the diagnosis of gluten allergy is based on prick tests and specific RAST tests.

Non-celiac gluten sensitivity, or more simply gluten sensitivity, is a recently identified pathological condition correlated with the intake of gluten. It manifests itself with both gastroenterological symptoms (bloating, diarrhoea and abdominal pain), and extra-enteric symptoms (headache, joint and muscle pains, blurred vision) which, in large part, are comparable to the symptoms of celiac disease or irritable bowel syndrome. However, unlike in the latter cases, a subject with gluten sensitivity does not show any atrophy of the intestinal villi, or an autoimmune response.

In London in 2011 there was the first Consensus Conference on gluten sensitivity, which, among other things, sought to give a definition to this condition. Unfortunately, the scant knowledge regarding the etiology of gluten sensitivity and the symptoms shared with other pathologies has also made it difficult to define this condition. In fact, there exists no definition of what gluten sensitivity is; rather, what it is not has been defined. In other words, at present, a subject is considered likely to be sensitive to gluten when he/she tests negative both for the specific serological markers of celiac disease, and the specific IgE of gluten allergy and, moreover, when an intestinal biopsy is within the normal range, i.e. without any villous atrophy.

Carroccio et al, 2012 discloses a double-blinded placebo controlled (DBPC) challenge with wheat to a patient who had been on a standard elimination diet.

DBPC was performed with capsules A or B containing wheat or xylose, respectively. Capsules A or B were given for two consecutive weeks and then after 1 week wash-out, the patients received the other capsules for another two weeks.

Therefore, at present it is possible to determine whether a subject is sensitive to gluten only by process of elimination.

However, there is a greatly felt need to define a protocol for determining gluten sensitivity in a subject, since approximately 6%-14% of the population suffers from this pathology and therefore the problem is highly relevant from an epidemiological standpoint.

The present invention has resolved the above-described technical problem with a kit for use in a method for determining gluten sensitivity as specified in the appended independent claims.

The preferred aspects of the present invention are specified in the appended dependent claims.

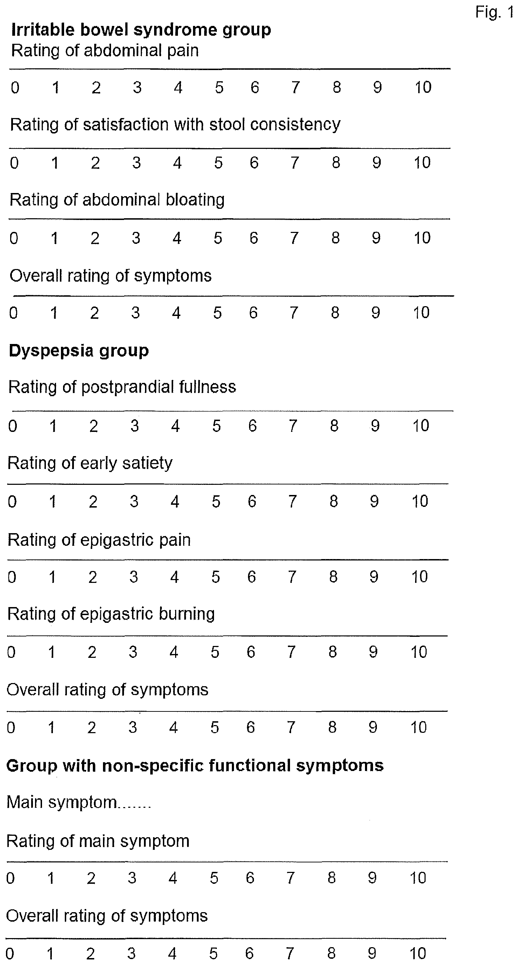

The present invention will be described below in detail, also with the aid of Figure 1, which refers to an example of an *ad hoc* questionnaire for collecting data regarding the health status of a subject who carries out the method of the invention.

A first aspect of the present invention refers to a kit comprising:
- a kit identification code;
- at least one oral formulation of gluten in a quantity comprised between 4 and 10 grams;
- at least one oral formulation of placebo in a quantity comprised between 4 and 10 grams;
said at least one oral formulation of gluten and at least one oral formulation of placebo being for double-blind administration and each being identified by a code,
for use in a diagnostic method for determining gluten sensitivity in a subject, said method comprising at least one step of:
- providing said subject with said kit;
- taking at least one of said oral formulation of gluten and at least one of said oral formulation of placebo in any order according to a double-blind protocol;
- collecting the data referring to the health status of said subject;
- determining the nature of each oral formulation taken and establish the chronology of intake; and
- evaluating whether a worsening in at least 30% of the data referring to the health status of said subject occurs during said at least one step of administering at least one oral formulation of gluten, thus concluding that the subject is sensitive to gluten.

According to a preferred embodiment of the invention, the number of said at least one oral formulation of gluten and the number of said at least one oral formulation of placebo is the same, preferably it is at least five, more preferably at least seven.

According to a further preferred embodiment of the invention, the at least one oral formulation is at least one pill, at least one pastille, at least one lozenge, or at least one tablet.

According to a further preferred embodiment of the invention, the placebo is rice starch.

According to a further preferred embodiment of the invention, the kit for use as disclosed above further comprises a questionnaire prepared *ad hoc* for the collection, preferably via Web, of data referring to the health status of said subject. Preferably, said questionnaire comprises at least one rating scale, preferably a verbal numerical scale (VNS), or a visual analogue scale (VAS) or a verbal rating scale (VRS).

According to a further preferred embodiment of the invention, the step of taking at least one of said oral formulation of gluten is performed before or after said step of taking at least one of said oral formulation of placebo. According to a further preferred embodiment of the invention, in the diagnostic method, at least one wash-out phase takes place between said step of taking at least one of said oral formulation of gluten and said step of taking at least one oral formulation of placebo.

According to a further preferred embodiment of the invention, in the diagnostic method, said step of taking at least one oral formulation of gluten, and/or said step of taking at least one oral formulation of placebo, and/or said at least one wash-out phase takes place over a period of at least five days, preferably at least seven days, more preferably for seven days.

According to a further preferred embodiment of the invention, the diagnostic method comprises at least one step wherein said subject takes at least one oral formulation of gluten or at least one oral formulation of placebo for a week; and/or at least one week-long wash-out phase wherein said subject takes neither the oral formulation of gluten, nor the oral formulation of placebo; and/or at least one step wherein said subject takes at least one oral formulation of placebo or at least one oral formulation of gluten for a week.

According to a further preferred embodiment of the invention, said subject has previously followed a gluten-free diet.

According to a further preferred embodiment of the invention, in the diagnostic method, said step of collecting data referring to the health status of the subject is performed by collecting said data in a questionnaire.

According to a further preferred embodiment of the invention, in the diagnostic method, said data collection step is performed by the subject at the start and at the end of said at least one step of taking at least one oral formulation of gluten and/or at the start and at the end of said at least one step of taking at least one oral formulation of placebo.

The kit that is provided to the subject for the purpose of carrying out the method of the present invention comprises (i) at least one oral formulation of gluten, i.e. a quantity of gluten comprised between 4 and 10 grams and formulated for oral administration, (ii) and at least one oral formulation of placebo, i.e. a quantity of placebo which ranges between 4 and 10 grams, wherein the gluten and the placebo are formulated for double-blind oral administration, i.e. the two oral formulations are identical in aesthetic appearance, that is, in form, but differ in substance, because one contains gluten and the other contains placebo. Moreover, each of the two formulations is identified by a code.

In this manner, the at least one oral formulation of gluten and the at least one oral formulation of placebo contained in a kit are such as to be indistinguishable by any subject. Moreover, the at least one oral formulation of gluten and the at least one oral formulation of placebo contained in the kit are univocally identified by any code whatsoever, for example a colour code, a numerical code, an alphabetic or alphanumeric code.

The correspondence of this code with the nature of the substance, i.e. whether it is an oral formulation containing gluten or an oral formulation containing placebo, is known only to the kit manufacturer. In fact, the kit is characterized by an identification code, based on which it is possible to determine the nature of the oral formulations that a subject has taken (i.e. gluten or placebo) because the manufacturer associated (at the time of production/sale) the identification code of the kit with each code of the oral formulations and hence the corresponding substances contained in each oral formulation (i.e. gluten or placebo). In this manner, it will be possible, after carrying out the method, to contact the manufacturer, for example by telephone or via web, specify the identification code of the kit, determine the nature of each oral formulation taken and establish the chronology of intake. In other words, it will be possible afterwards to distinguish the nature of the substance taken. In fact, the at least one oral formulation of gluten and the at least one oral formulation of placebo are identified by a code with the aim of enabling the correct association by the manufacturer. For example, the manufacturer can produce a kit with a certain identification code X in which the oral formulation of gluten has a code A, whereas the oral formulation of placebo in the same kit has a code B. The manufacturer records, in a database, that the kit having the identification code X has an oral formulation of gluten which corresponds to code A and an oral formulation of placebo which corresponds to code B. In all other respects the oral formulations are identical, except in content, which is obviously not visible. At the end of the period of intake, the subject will contact the manufacturer in order to associate, via the identification code of the kit, the code of each oral formulation with the substance contained therein and consequently establish the chronology of the intake, that is, if he/she took the gluten first and then the placebo or vice versa.

In the context of the present invention, the term "double blind" means that neither the subject, nor whoever suggests carrying out the method (generally a physician) is aware of when gluten or the placebo is taken. Said at least one oral formulation of gluten and said at least one oral formulation of placebo are preferably in the form of pastilles, pills, capsules or tablets.

Preferably, each kit comprises at least one, preferably at least five, more preferably at least seven pastilles, pills, capsules or tablets of gluten and at least one, preferably at least five, more preferably at least seven pastilles, pills, capsules or tablets of placebo. Preferably, the number of the oral formulations of gluten and the number of oral formulations of placebo for each kit is the same.

In fact, the step of taking at least one oral formulation of gluten and the step of taking at least one oral formulation of placebo on a double-blind basis comprises at least one intake, preferably at least seven intakes. Preferably, said at least one step of taking at least one oral formulation of gluten is carried out before or after said at least one step of taking at least one oral formulation of placebo.

Therefore, in particular, the method of determining gluten sensitivity according to the present invention envisages that the subject takes gluten for a week and takes the placebo for a week or vice-versa; the intake of gluten and/or placebo takes place randomly, on a double-blind basis. Preferably, a wash-out phase takes place between said at least one step of taking at least one oral formulation of gluten and said at least one step of taking at least one oral formulation of placebo. Said wash-out phase preferably lasts for at least five days, preferably for at least seven days, more preferably for seven days.

In the context of the present invention, the term wash-out means a period between two periods of taking gluten or placebo in which the subject takes nothing and should thus "expel" everything he/she took in previously.

The particularly preferred embodiment of the method according to the present invention envisages: at least one step of a subject taking at least one oral formulation of gluten or at least one of said oral formulation of placebo for a week; and/or at least a wash-out phase of one week in which said subject takes neither the oral gluten formulation nor the oral formulation of placebo; and/or at least one step in which said subject takes at least one oral formulation of placebo or at least one of said oral formulation of gluten for a week.

Alternatively, the subject can take the placebo for a week, then undergo a week of wash-out and finally take gluten for another week. The intake step is carried out randomly on a double-blind basis, so the subject does not know which substance he/she is taking.

Said placebo is preferably selected from among: rice starch and other inert molecules, possibly also in different dosages by weight.

According to a preferred embodiment, the subject to whom the method of the invention is applied previously followed a gluten-free diet.

The step of collecting data regarding the health status of the subject is preferably carried out by collecting said data in a questionnaire. Said questionnaire is prepared *ad hoc* to collect data regarding the health status of a subject who carries out the method of the invention. In particular, said questionnaire is a standard form which includes questions related to the health status of said subject. The subject can respond using the evaluation scales associated with each question. The evaluation scale is preferably a verbal numerical scale (VNS), or a visual analogue scale (VAS) or a verbal rating scale (VRS).

Preferably, data is collected by the subject via Web at the beginning and at the end of said at least one step of taking at least one oral formulation of gluten and/or at the start and at the end of at least one step of taking at least one oral formulation of placebo.

In particular, the questionnaire can contain questions about the health status of the subject which are related to the group to which the subject belongs. In particular, subjects can be divided into groups based on their symptoms. For example, the subjects who carry out the method according to the present invention can be divided into a group with irritable bowel syndrome or a group with symptoms of a dyspeptic, diarrhoeic type, with alterations of the bowel or extraintestinal manifestations such as mild neurological or dermatological manifestations, or into a group that exhibits non-specific symptoms.

The questions can be specific for each group.

An example of a questionnaire is shown in Figure 1, where different questions are proposed for the various groups of subjects with a numerical rating scale in which the subject will indicate a value corresponding to the specific health status required in the question.

Applying the proposed method, it has been observed that a worsening in at least 30% of the data regarding a subject's health status, in particular the main symptoms, occurring during said at least one step of administering at least one oral formulation of gluten, makes it possible to say that the subject is sensitive to gluten.

Therefore, a further aspect of the present invention relates to said kit for use in a diagnostic method to determine gluten sensitivity in a subject. Preferably, the determination of gluten sensitivity in said subject is achieved by applying the method of the present invention.

A further aspect of the present disclosure regards a kit for carrying out the method to determine gluten sensitivity according to the present invention, said kit comprising:
- a kit identification code;
- at least one oral formulation of gluten in a quantity comprised between 4 and 10 grams;
- at least one oral formulation of placebo in a quantity comprised between 4 and 10 grams;
said oral formulation of gluten and said oral formulation of placebo being for double-blind administration and each being identified by a code. Preferably, said at least one oral formulation of gluten and said at least one oral formulation of placebo is at least five, preferably seven formulations. In a particularly preferred embodiment, the number of oral formulations of said gluten is equal to the number of oral formulations of said placebo. For example, the kit comprising at least seven oral formulations of gluten and at least seven oral formulations of placebo is useful, for example, for enabling a weekly intake of said oral formulations. According to a preferred embodiment of the invention, the at least one oral formulation consists of at least one pill, at least one pastille, at least one lozenge, or at least one tablet.

Said oral formulations are identified by a code, for example a colour code, a numerical code, an alphabetic code, or an alphanumeric code. For the purpose of the method, said code will serve to understand when gluten was taken and when the placebo was taken, as earlier described. According to a further embodiment of the present invention, the kit further comprises an *ad hoc* questionnaire prepared to collect data regarding the health status of a subject who carries out the method of the invention.

In particular, said questionnaire is a standard form containing questions related to the health status of said subject. The subject can answer using the evaluation scale associated with each question. The evaluation scale is preferably a verbal numerical scale (VNS), a visual analogue scale (VAS) or a verbal rating scale (VRS).

## Claims

1. A kit comprising:
- a kit identification code;
- at least one oral formulation of gluten in a quantity comprised between 4 and 10 grams;
- at least one oral formulation of placebo in a quantity comprised between 4 and 10 grams;
said at least one oral formulation of gluten and at least one oral formulation of placebo being for double-blind administration and each being identified by a code,
for use in a diagnostic method for determining gluten sensitivity in a subject, said method comprising at least one step of:
- providing said subject with said kit;
- taking at least one of said oral formulation of gluten and at least one of said oral formulation of placebo in any order according to a double-blind protocol;
- collecting the data referring to the health status of said subject;
- determining the nature of each oral formulation taken and establish the chronology of intake; and
- evaluating whether a worsening in at least 30% of the data referring to the health status of said subject occurs during said at least one step of administering at least one oral formulation of gluten, thus concluding that the subject is sensitive to gluten.

2. The kit for use according to claim 1, wherein the number of said at least one oral formulation of gluten and the number of said at least one oral formulation of placebo is the same, preferably it is at least five, more preferably at least seven.

3. The kit for use according to claim 1 or 2, wherein said at least one oral formulation is at least one pill, at least one pastille, at least one lozenge, or at least one tablet.

4. The kit for use according to any one of claims 1-3, wherein said placebo is rice starch.

5. The kit for use according to any one of claims 1-4, further comprising a questionnaire prepared *ad hoc* for the collection, preferably via Web, of data referring to the health status of said subject.

6. The kit for use according to claim 5, wherein said questionnaire comprises at least one rating scale, preferably a verbal numerical scale (VNS), or a visual analogue scale (VAS) or a verbal rating scale (VRS).

7. The kit for use according to any of the preceding claims, wherein, in the diagnostic method, said step of taking at least one of said oral formulation of gluten is performed before or after said step of taking at least one of said oral formulation of placebo.

8. The kit for use according to any of the preceding claims, wherein, in the diagnostic method, at least one wash-out phase takes place between said step of taking at least one of said oral formulation of gluten and said step of taking at least one oral formulation of placebo.

9. The kit for use according to any of the preceding claims, wherein, in the diagnostic method, said step of taking at least one oral formulation of gluten, and/or said step of taking at least one oral formulation of placebo, and/or said at least one wash-out phase takes place over a period of at least five days, preferably at least seven days, more preferably for seven days.

10. The kit for use according to any of the preceding claims, wherein the diagnostic method comprises at least one step wherein said subject takes at least one oral formulation of gluten or at least one oral formulation of placebo for a week; and/or at least one week-long wash-out phase wherein said subject takes neither the oral formulation of gluten, nor the oral formulation of placebo; and/or at least one step wherein said subject takes at least one oral formulation of placebo or at least one oral formulation of gluten for a week.

11. The kit for use according to any of the preceding claims, wherein said subject has previously followed a gluten-free diet.

12. The kit for use according to any of the preceding claims, wherein, in the diagnostic method, said step of collecting data referring to the health status of the subject is performed by collecting said data in a questionnaire.

13. The kit for use according to claim 12, wherein said questionnaire is a standard form containing questions about the health status of a subject, wherein said questions are associated with a rating scale, preferably a verbal numerical scale (VNS), or a visual analogue scale (VAS) or a verbal rating scale (VRS).

14. The kit for use according to any of the preceding claims, wherein, in the diagnostic method, said data collection step is performed by the subject at the start and at the end of said at least one step of taking at least one oral formulation of gluten and/or at the start and at the end of said at least one step of taking at least one oral formulation of placebo.

## Patentansprüche

1. Kit umfassend:
- ein Kitidentifikationscode;
- mindestens eine orale Formulierung von Gluten in einer Menge zwischen 4 und 10 Gramm;
- mindestens eine orale Formulierung von Placebo in einer Menge zwischen 4 und 10 Gramm;
wobei mindestens eine orale Formulierung von Gluten und mindestens eine orale Formulierung von Placebo für die Doppelblind-Verabreichung bestimmt sind und wobei jeweils durch einen Code identifiziert werden,
zur Verwendung in einem diagnostischen Verfahren zur Bestimmung der Glutenempfindlichkeit in einem Subjekt, wobei das Verfahren mindestens einen Schritt umfasst:
- Anbieten dem Subjekt das Kit;
- Einnehmen mindestens einer oralen Formulierung von Gluten und mindestens einer oralen Formulierung von Placebo in einer beliebigen Reihenfolge nach einem Doppelblindprotokoll;
- Sammeln der Daten, die sich auf den Gesundheitszustand des Subjekts beziehen;
- Bestimmen der Art jeder eingenommenen oralen Formulierung und Festlegen der Chronologie der Einnahme; und
- Beurteilen, ob eine Verschlimmerung von mindestens 30% der Daten bezogen auf den Gesundheitszustand des Subjekts während des mindestens einen Schritts der Verabreichung mindestens einer oralen Formulierung von Gluten auftritt, was zur Schlussfolgerung führt, dass das Subjekt auf Gluten empfindlich ist.

2. Kit zur Verwendung nach Anspruch 1, wobei die Anzahl der mindestens einen oralen Formulierung von Gluten und die Anzahl der mindestens einen oralen Formulierung von Placebo die gleiche ist, vorzugsweise ist sie mindestens fünf, besonders bevorzugt mindestens sieben.

3. Kit zur Verwendung nach Anspruch 1 oder 2, wobei die mindestens eine orale Formulierung mindestens eine Pille, mindestens eine Pastille, mindestens eine Lutschtablette oder mindestens eine Tablette ist.

4. Kit zur Verwendung nach einem der Ansprüche 1 bis 3, wobei das Placebo Reisstärke ist.

5. Kit zur Verwendung nach einem der Ansprüche 1 bis 4, ferner umfassend einen ad hoc erstellten Fragebogen für die Sammlung, vorzugsweise über das Web, von Daten, die sich auf den Gesundheitszustand des Subjekts beziehen.

6. Kit zur Verwendung nach Anspruch 5, wobei der Fragebogen mindestens eine Bewertungsskala, vorzugsweise eine verbale numerische Skala (VNS) oder eine visuelle Analogskala (VAS) oder eine verbale Bewertungsskala (VRS) umfasst.

7. Kit zur Verwendung nach einem der vorhergehenden Ansprüche, wobei im diagnostischen Verfahren der Schritt der Einnahme von mindestens einer der oralen Formulierung von Gluten vor oder nach dem Schritt der Einnahme von mindestens einer der oralen Formulierung von Placebo vorgenommen wird.

8. Kit zur Verwendung nach einem der vorhergehenden Ansprüche, wobei im diagnostischen Verfahren mindestens eine Auswaschphase zwischen dem Schritt der Einnahme von mindestens einer der oralen Formulierung von Gluten und dem Schritt der Einnahme von mindestens einer der oralen Formulierung von Placebo stattfindet.

9. Kit zur Verwendung nach einem der vorhergehenden Ansprüche, wobei im diagnostischen Verfahren der Schritt der Einnahme von mindestens einer oralen Formulierung von Gluten und/oder der Schritt der Einnahme von mindestens einer oralen Formulierung von Placebo und/oder mindestens eine Auswaschphase über einen Zeitraum von mindestens fünf Tagen, vorzugsweise mindestens sieben Tagen, besonders bevorzugt über sieben Tage stattfindet.

10. Kit zur Verwendung nach einem der vorhergehenden Ansprüche, wobei das diagnostische Verfahren mindestens einen Schritt umfasst, wobei das Subjekt mindestens eine orale Formulierung von Gluten oder mindestens eine orale Formulierung von Placebo für eine Woche einnimmt; und/oder mindestens eine einwöchige Auswaschphase, wobei das Subjekt weder die orale Formulierung von Gluten noch die orale Formulierung von Placebo einnimmt; und/oder mindestens einen Schritt, wobei das Subjekt mindestens eine orale Formulierung von Placebo oder mindestens eine orale Formulierung von Gluten für eine Woche einnimmt.

11. Kit zur Verwendung nach einem der vorhergehenden Ansprüche, wobei das Subjekt zuvor einer glutenfreien Diät gefolgt ist.

12. Kit zur Verwendung nach einem der vorhergehenden Ansprüche, wobei im Diagnoseverfahren der Schritt des Sammelns von Daten, die sich auf den Gesundheitszustand des Subjekts beziehen, durch Sammeln der Daten in einem Fragebogen durchgeführt wird.

13. Kit zur Verwendung nach Anspruch 12, wobei der Fragebogen ein Standardformular ist, der Fragen über den Gesundheitszustand eines Subjekts enthält, wobei die Fragen mit einer Bewertungsskala, vorzugsweise einer verbalen numerischen Skala (VNS) oder einer visuellen Analogskala (VAS) oder einer verbalen Ratingskala (VRS) verbunden sind.

14. Kit zur Verwendung nach einem der vorhergehenden Ansprüche, wobei im diagnostischen Verfahren der Datensammelschritt vom Subjekt zu Beginn und am Ende des mindestens einen Schritts der Einnahme von mindestens einer oralen Formulierung von Gluten und/oder zu Beginn und am Ende des mindestens einen Schritts der Einnahme von mindestens einer oralen Formulierung von Placebo durchgeführt wird.

## Revendications

1. Kit comprenant :
- un code d'identification de kit ;
- au moins une formulation orale de gluten dans une quantité comprise entre 4 et 10 grammes ;
- au moins une formulation orale de placebo dans une quantité comprise entre 4 et 10 grammes ;
lesdites au moins une formulation orale de gluten et au mois une formulation orale de placebo étant destinées à une administration à double insu et chacune étant identifiée par un code,
pour l'utilisation dans une méthode de diagnostic destinée à déterminer la sensibilité au gluten chez un sujet, ladite méthode comprenant au moins une étape de :
- fournir ledit kit au dit sujet ;
- prendre au moins une de ladite formulation orale de gluten et au moins une de ladite formulation orale de placebo dans n'importe quel ordre selon un protocole à double insu ;
- recueillir les données en référence à l'état de santé dudit sujet ;
- déterminer la nature de chaque formulation orale prise et établir la chronologie d'apport ; et
- évaluer si une évaluation défavorable dans au moins 30 % des données en référence à l'état de santé dudit sujet a lieu lors de ladite au moins une étape consistant à administrer au moins une formulation orale de gluten, pour ainsi en conclure que le sujet est sensible au gluten.

2. Kit pour utilisation selon la revendication 1, dans lequel le nombre de ladite au moins une formulation orale de gluten et le nombre de ladite au moins une formulation orale de placebo est le même, de préférence celui-ci est au moins de cinq, idéalement d'au moins sept.

3. Kit pour utilisation selon la revendication 1 ou 2, dans lequel ladite au moins une formulation orale est au moins une pilule, au moins une pastille, au moins un comprimé ou au moins un cachet.

4. Kit pour utilisation selon l'une quelconque des revendications de 1 à 3, dans lequel ledit placebo est de l'amidon de riz.

5. Kit pour utilisation selon l'une quelconque des revendications de 1 à 4, comprenant de plus un questionnaire ad hoc pour la collecte, de préférence via Internet, des données en référence à l'état de santé dudit sujet.

6. Kit pour utilisation selon la revendication 5, dans lequel ledit questionnaire comprend au moins une échelle d'évaluation, de préférence une échelle verbale numérique (EVN) ou une échelle visuelle analogue (EVA) ou une échelle d'évaluation verbale (EEV).

7. Kit pour utilisation selon l'une quelconque des revendications précédentes, dans lequel, dans la méthode de diagnostic, ladite étape de prise d'au moins une de ladite formulation orale de gluten est réalisée avant ou après ladite étape de prise d'au moins une de ladite formulation orale de placebo.

8. Kit pour utilisation selon l'une quelconque des revendications précédentes, dans lequel, dans la méthode de diagnostic, au moins une phase de parenthèse thérapeutique a lieu entre ladite étape de prise d'au moins une de ladite formulation orale de gluten et ladite étape de prise d'au moins une formulation orale de placebo.

9. Kit pour utilisation selon l'une quelconque des revendications précédentes, dans lequel, dans la méthode de diagnostic, ladite étape de prise d'au moins une formulation orale de gluten et/ou ladite étape de prise d'au moins une formulation orale de placebo et/ou ladite au moins une phase de parenthèse thérapeutique a lieu sur une période d'au moins cinq jours, de préférence au moins sept jours, idéalement pendant sept jours.

10. Kit pour utilisation selon l'une quelconque des revendications précédentes, dans lequel la méthode de diagnostic comprend au moins une étape dans laquelle ledit sujet prend au moins une formulation orale de gluten ou au moins une formulation orale de placebo pendant une semaine ; et/ou au moins une phase de parenthèse thérapeutique d'une semaine dans laquelle ledit sujet prend soit la formulation orale de gluten soit la formulation orale de placebo ; et/ou au moins une étape dans laquelle ledit sujet prend au moins une formulation orale de placebo ou au moins une formulation orale de gluten pendant une semaine.

11. Kit pour utilisation selon l'une quelconque des revendications précédentes, dans lequel ledit sujet a précédemment suivi un régime sans gluten.

12. Kit pour utilisation selon l'une quelconque des revendications précédentes, dans lequel, dans la méthode de diagnostic, ladite étape de collecte des données en référence à l'état de santé du sujet est réalisée en recueillant lesdites données dans un questionnaire.

13. Kit pour utilisation selon la revendication 12, dans lequel ledit questionnaire est un formulaire standard contenant des questions sur l'état de santé d'un sujet, dans lequel lesdites questions sont associées à une échelle d'évaluation, de préférence une échelle verbale numérique (EVN) ou une échelle visuelle analogue (EVA) ou une échelle d'évaluation verbale (EEV) .

14. Kit pour utilisation selon l'une quelconque des revendications précédentes, dans lequel, dans la méthode de diagnostic, ladite étape de collecte des données est réalisée par le sujet au début et à la fin de ladite au moins une étape de prise d'au moins une formulation orale de gluten et/ou au début et à la fin de ladite au moins une étape de prise d'au moins une formulation orale de placebo.
